# EUROPEAN PATENT APPLICATION

(11) **EP 4 480 425 A1**
(43) Date of publication of application: **25.12.2024**
(21) Application number: 23752827.8
(22) Date of filing: 06.02.2023
(51) Int. Cl.: A61B 17/00, A61B 17/32

(54) **ULTRASONIC TREATMENT TOOL**

(30) Priority: 14.02.2022 JP 2022020291
(71) Applicant: FUJIFILM Corporation, Tokyo 106-8620 (JP)
(72) Inventor: HIGASHI, Kohei, Ashigarakami-gun, Kanagawa 258-8577 (JP); NAKAMURA, Koichiro, Ashigarakami-gun, Kanagawa 258-8577 (JP)
(74) Representative: HGF
(86) International application number: PCT/JP2023/003798
(87) International publication number: WO 2023/153363

(57) **Abstract**

Provided is an ultrasonic treatment tool that can efficiently incise a biological tissue.

An ultrasonic treatment tool includes a grip part (15) that is provided at a distal end part and that grips a biological tissue in a subject, an ultrasound oscillator (16) that transmits ultrasonic oscillation to the biological tissue gripped by the grip part (15), and a stress concentration structure (37) that is provided at the grip part (15) and that protrudes from a grip surface (32A) facing the biological tissue, in which the stress concentration structure (37) is a long protrusion disposed along a longitudinal direction of the grip part (15), and a distal end part (37A) from the grip surface (32A) in a protruding direction is formed in a continuous sawtooth shape.

## Description

### BACKGROUND OF THE INVENTION

### 1. Field of the Invention

The present invention relates to an ultrasonic treatment tool used in sealing and incising a biological tissue such as a blood vessel.

### 2. Description of the Related Art

In a medical field, it is known that various types of treatment are performed on a subject by using an ultrasonic treatment tool that generates ultrasonic oscillation. JP2016-511096A describes an ultrasonic treatment tool that comprises a pair of grip parts that grips a biological tissue such as a blood vessel and an ultrasound oscillator (ultrasonic transducer), in which ultrasonic oscillation is transmitted via the grip parts, and treatment such as sealing and incision of the biological tissue is performed. The ultrasound oscillator is composed of various types of devices such as a piezoelectric material and converts supplied power into ultrasonic oscillation.

In addition, in the ultrasonic treatment tool described in JP2016-511096A, a grip surface of the grip part, which faces the biological tissue, has a plurality of saw teeth. The plurality of saw teeth are disposed with predetermined intervals placed. Accordingly, a gripping property with respect to the biological tissue in a case where the ultrasonic treatment tool grips the biological tissue improves.

### SUMMARY OF THE INVENTION

Since the gripping property of gripping the biological tissue is improved, the ultrasonic treatment tool described in JP2016-511096A is suitable for treatment of sealing the biological tissue, but is not suitable for treatment of incising the biological tissue. That is, in the ultrasonic treatment tool disclosed in JP2016-511096A, since the plurality of saw teeth are disposed with predetermined intervals placed, a portion without the saw teeth has a very small pressure applied to the biological tissue compared to a portion with the saw teeth. Accordingly, even in a case where a force of gripping the biological tissue is large, the biological tissue cannot be incised in the portion without the saw teeth in some cases.

An object of the present invention is to provide an ultrasonic treatment tool that can efficiently perform treatment of incising a biological tissue.

According to an aspect of the present invention, there is provided an ultrasonic treatment tool comprising a grip part, an ultrasound oscillator, and a stress concentration structure, in which the stress concentration structure is a long protrusion disposed along a longitudinal direction of the grip part, and a distal end part from the grip surface in a protruding direction is formed in a continuous sawtooth shape. The grip part is provided at a distal end part and grips a biological tissue in a subject. The ultrasound oscillator transmits ultrasonic oscillation to the biological tissue gripped by the grip part. The stress concentration structure is provided at the grip part and protrudes from a grip surface facing the biological tissue.

It is preferable that the ultrasound oscillator is disposed at the grip part. It is preferable that the stress concentration structure has hardness of 40 HBW or more in Brinell hardness. It is preferable that a surface of the grip part, which faces the stress concentration structure, has hardness of 40 HBW or more in Brinell hardness.

It is preferable that the ultrasound oscillator is composed of a piezoelectric material and is driven by an alternating current voltage signal having a frequency at which a difference from a resonance frequency corresponding to a thickness dimension of the piezoelectric material is within 20%. It is preferable that the piezoelectric material is lead zirconate titanate. It is preferable that a Curie point of the piezoelectric material is 200°C or more.

It is preferable that an alternating current voltage signal for driving the ultrasound oscillator is continuously supplied. It is preferable that the grip part grips a blood vessel which is the biological tissue.

It is preferable that the ultrasonic treatment tool is a surgical treatment tool comprising an insertion part that is installed consecutively to a base end of the grip part and an operating part that is installed consecutively to a base end of the insertion part. It is preferable that the grip part comprises a pair of grip pieces that grips the biological tissue, and the stress concentration structure is provided at one grip piece.

With the present invention, the ultrasonic treatment tool that can efficiently perform treatment of incising the biological tissue can be provided.

### BRIEF DESCRIPTION OF THE DRAWINGS

Fig. 1 is an explanatory view showing a configuration of an ultrasonic treatment tool system.
Figs. 2A and 2B are cross sectional views of main portions of an ultrasonic treatment tool and are cross sectional views of the main portions showing a closed state (Fig. 2A) and an open state (Fig. 2B) of a grip part.
Fig. 3 is a perspective view of the ultrasonic treatment tool in a state where the grip part is closed.
Fig. 4 is a perspective view of the ultrasonic treatment tool in a state where the grip part is open.
Fig. 5 is a cross sectional view of main portions cut along line V-V of Fig. 2A.
Fig. 6 is an explanatory view showing a state where treatment is performed on a blood vessel gripped by the grip part.
Fig. 7 is a perspective view of an edge part of a stress concentration structure.
Fig. 8 is a block diagram showing an outline of an ultrasonic drive unit.
Fig. 9 is a graph showing a relationship between a driving time of an ultrasound oscillator and a blood vessel temperature in a case where the blood vessel is gripped and the ultrasound oscillator is driven.
Figs. 10A to 10C are explanatory views showing operations in a case where treatment of sealing the blood vessel is performed with the ultrasonic treatment tool.
Figs. 11A to 11C are explanatory views showing operations in a case where treatment of incising the blood vessel is performed with the ultrasonic treatment tool.
Fig. 12 is a cross sectional view of main portions showing a configuration of a grip part in a second embodiment.
Fig. 13 is an explanatory view showing configurations of a stress concentration structure and a facing member in the second embodiment.
Fig. 14 is an explanatory view showing some of an operation in a case where treatment of incising a blood vessel is performed in the second embodiment.
Fig. 15 is a cross sectional view of main portions showing a configuration of a grip part in a modification example.

### DESCRIPTION OF THE PREFERRED EMBODIMENTS

### [First Embodiment]

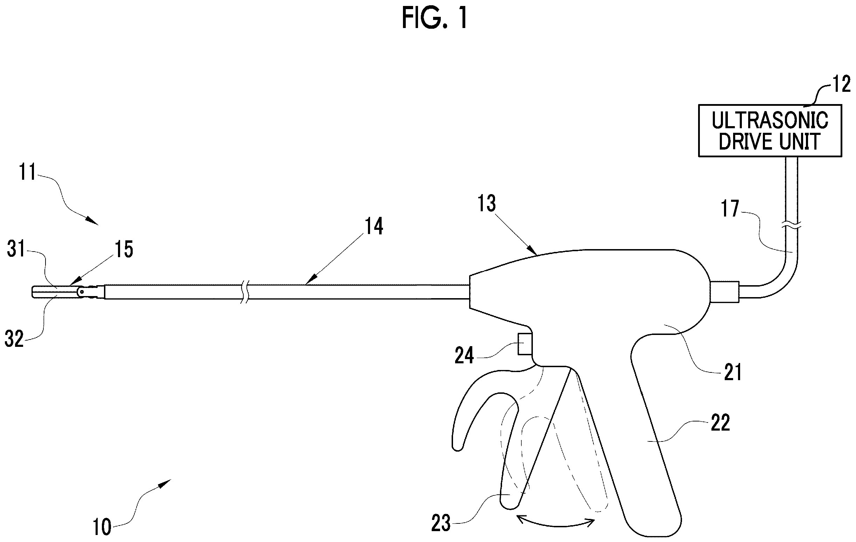

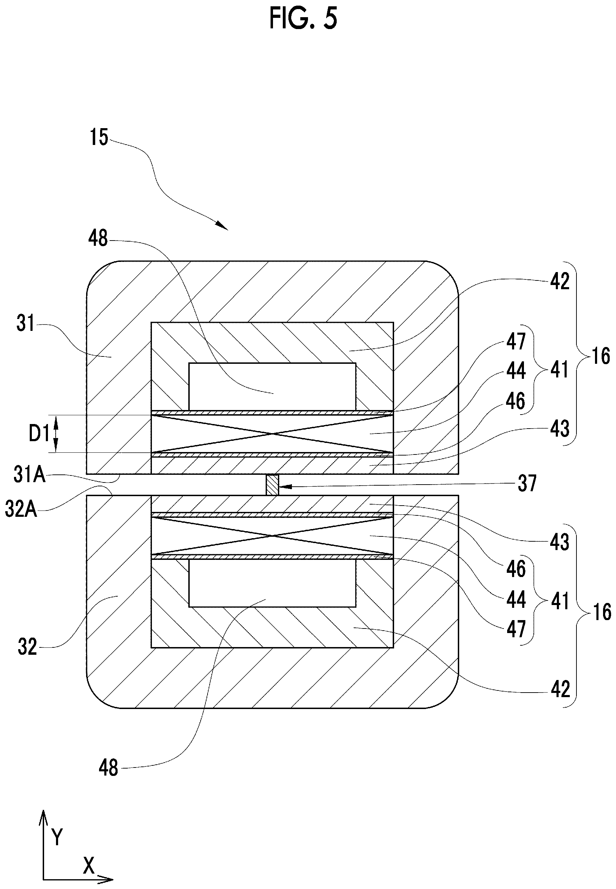

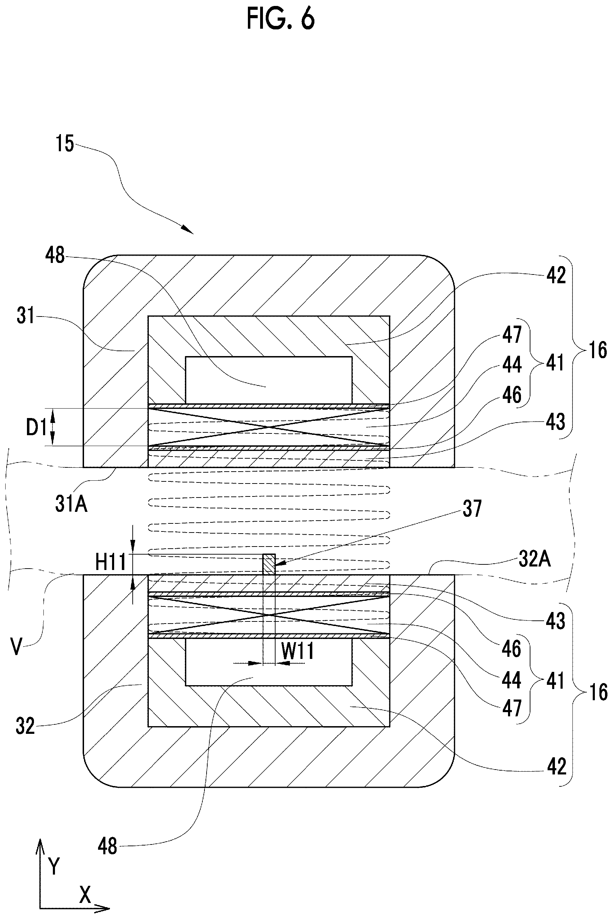

As shown in Fig. 1, an ultrasonic treatment tool system 10 comprises an ultrasonic treatment tool 11 and an ultrasonic drive unit 12. The ultrasonic treatment tool 11, which is a surgical treatment tool, comprises an operating part 13, an insertion part 14, a grip part 15, an ultrasonic oscillator unit 16 (see Figs. 5, 6, and the like), and a cable 17.

The operating part 13 comprises an operating part main body 21, a grip 22, a trigger 23, and an activation button 24. The operating part main body 21 is installed consecutively to a base end part of the insertion part 14. The operating part main body 21 is electrically connected to the ultrasonic drive unit 12 via the cable 17. The grip 22 is provided integrally with the operating part main body 21 and is held by a user in a case where the user performs a hand technique.

Although details thereof will be described later, the trigger 23 is provided to be swingable in a direction toward the grip 22 and a direction separated away from the grip 22 in order to open and close the grip part 15. The activation button 24 is connected to the cable 17 via a signal cable (not shown) or the like. In a case where the ultrasonic drive unit 12 is connected to the cable 17, an activation signal output in response to a pressing operation of the activation button 24 is input into a control unit 55 of the ultrasonic drive unit 12 to be described later.

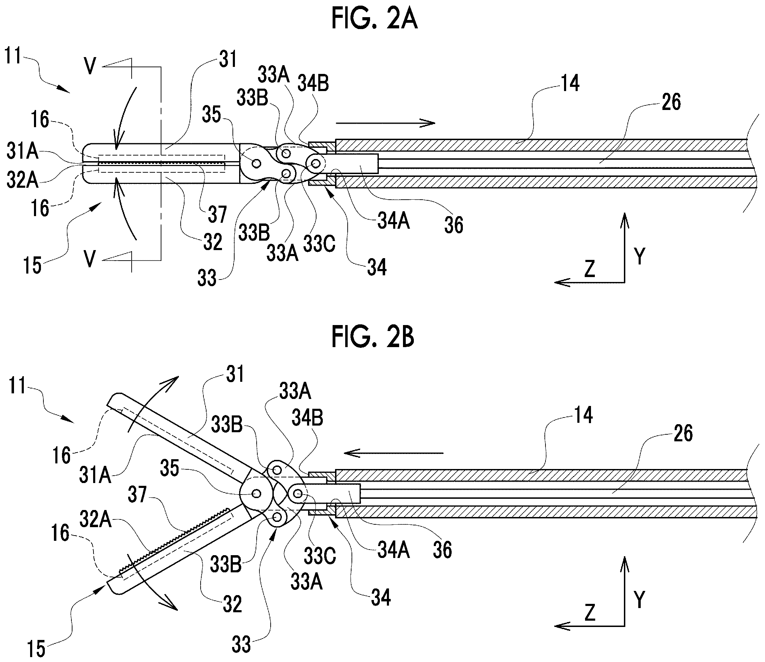

The insertion part 14 is installed consecutively to a base end part of the grip part 15. The insertion part 14 has a long cylindrical shape and is formed of, for example, a resin. In a case of sealing and incising a biological tissue, the insertion part 14 is inserted into a body (subject) of a patient, for example, via a trocar. An operation wire 26 (see Figs. 2A and 2B) is inserted into the insertion part 14. The operation wire 26 is provided integrally with the grip part 15. A base end part of the operation wire 26 is connected to the trigger 23 inside the operating part main body 21. For this reason, the operation wire 26 is operated to be pushed and pulled in an axial direction thereof in the insertion part 14 in response to swinging of the trigger 23. The trigger 23 is formed in a shape that is easy to operate in each of the direction toward the grip 22 and the direction separated away from the grip 22, for example, a shape branched in a Y-shape.

As shown in Figs. 2A and 2B, the grip part 15 is provided at a distal end part of the insertion part 14, that is, a distal end part of the ultrasonic treatment tool 11. The grip part 15 comprises a pair of grip pieces 31 and 32 that is vertically disposed, a link mechanism 33, and a support member 34 that supports the grip pieces 31 and 32 and the support member 34.

The ultrasonic oscillator unit 16 (see Figs. 5 and 6) is provided at each of the pair of grip pieces 31 and 32. The ultrasonic oscillator unit 16 is disposed on grip surfaces 31A and 32A of the grip pieces 31 and 32. The grip surfaces 31A and 32A are inner surfaces of the grip pieces 31 and 32 and are surfaces that face each other and that face the biological tissue. In a case where the grip part 15 is closed, the grip surfaces 31A and 32A of the pair of grip pieces 31 and 32 are near each other. Accordingly, the biological tissue can be gripped by being pinched between the pair of grip pieces 31 and 32.

The ultrasonic oscillator units 16 are in contact with the biological tissue gripped by the pair of grip pieces 31 and 32. An ultrasound oscillator 41 (see Figs. 5 and 6) constituting each ultrasonic oscillator unit 16 ultrasonically oscillates with power supplied from the ultrasonic drive unit 12 and causes a temperature rise attributable to the device driving (driving of the ultrasound oscillator 41). Accordingly, the ultrasonic oscillator units 16 transmit ultrasonic oscillation and heat attributable to the device driving to the biological tissue gripped by the pair of grip pieces 31 and 32.

In a case where the ultrasound oscillator is disposed at the operating part as in the ultrasonic treatment tool of the related art, a transmitting member having high stiffness needs to be provided between the operating part and the grip part in order to transmit ultrasonic oscillation, and the insertion part also needs to have high stiffness in order to support the transmitting member. On the other hand, in the present invention, since the ultrasound oscillator 41 is provided at the grip part 15 as described above, it is sufficient that a signal cable (not shown) for supplying power to the ultrasound oscillator 41 is provided between the operating part 13 and the grip part 15, that is, in the insertion part 14, and the insertion part 14 does not require high stiffness. That is, as the insertion part 14, it is possible to select an insertion part made of a material having more flexibility and/or an insertion part with a smaller outer diameter than the ultrasonic treatment tool of the related art, and thereby a degree of freedom in design improves. In addition, it is also possible that the insertion part 14 comprises a mechanism that deforms the insertion part 14 from a straight state into a bent state.

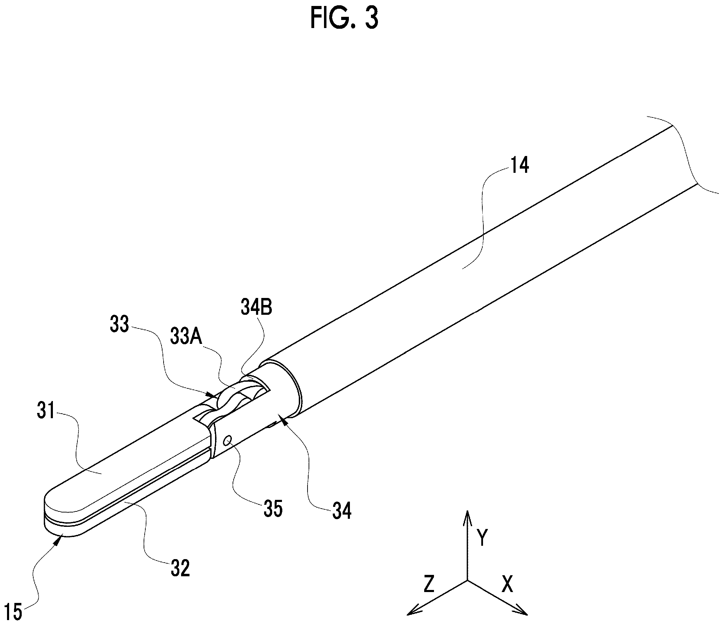

The pair of grip pieces 31 and 32 constituting the grip part 15 is openable and closable in an up-down direction about a support shaft 35. The support shaft 35 is supported by the support member 34. The grip pieces 31 and 32 are formed in a long plate shape, that is, a long plate shape having a dimension in an insertion direction Z longer than a dimension in a width direction X (see Fig. 3) and are formed in the same outer shape.

The link mechanism 33 comprises a link plate 33A, a connecting pin 33B, and a fitting pin 33C. One end part of the link plate 33A is connected to the pair of grip pieces 31 and 32 via the connecting pin 33B. Positions where the pair of grip pieces 31 and 32 is connected to the link plate 33A are at base end parts of the grip pieces 31 and 32 and are positions on a base end side of the support shaft 35. The other end part of the link plate 33A is connected to a connecting member 36 provided at a distal end of the operation wire 26 via the fitting pin 33C. The fitting pin 33C connects the link plate 33A to the connecting member 36 in a rotationally movable manner.

The connecting member 36 is formed in a cylindrical shape. The connecting member 36 penetrates through a through-hole 34A formed in the support member 34, and a part thereof is positioned inside the insertion part 14. The support member 34 is formed in a substantially cylindrical shape and is fixed to a distal end of the insertion part 14. The support member 34 has a notch 34B notched from a distal end thereof. Since the pair of grip pieces 31 and 32 and the link plate 33A move inside the notch 34B, the support member 34 does not hinder movement of the grip pieces 31 and 32 and the link plate 33A.

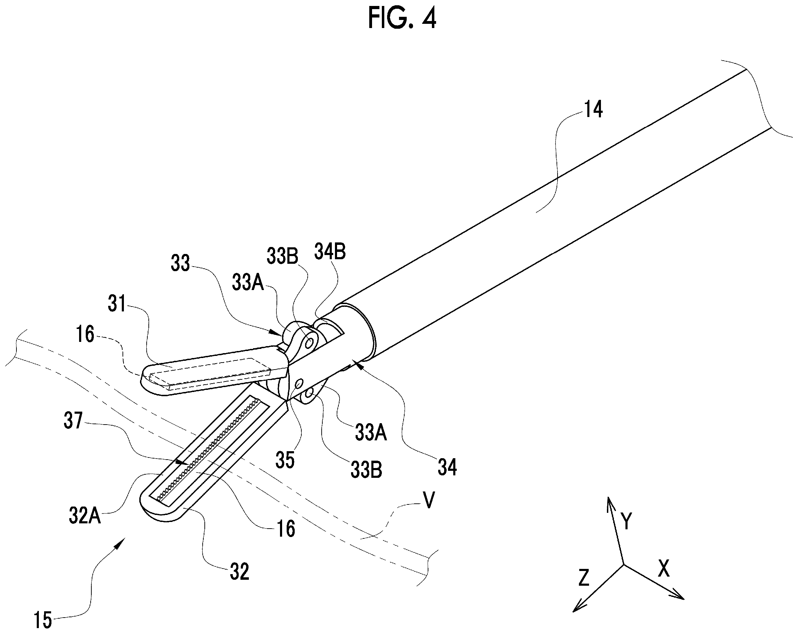

The link mechanism 33 converts linear motion caused by a pushing and pulling operation of the operation wire 26 into rotational motion and operates the pair of grip pieces 31 and 32 to be opened and closed. In the operating part main body 21, a cam mechanism (not shown) or the like that pulls the operation wire 26 to the base end side in a case where the trigger 23 is pulled to a grip 22 side and that conversely pushes out the operation wire 26 to a distal end side in a case where the trigger 23 is separated away from the grip 22 is provided. Accordingly, in a case where the trigger 23 is pulled to the grip 22 side, the grip pieces 31 and 32 are closed, and the grip part 15 is in a closed state (see Fig. 2A and Fig. 3), and in a case where the trigger 23 is separated away from the grip 22, the grip pieces 31 and 32 are opened and the grip part 15 is in an open state (see Fig. 2B and Fig. 4).

A stress concentration structure 37 is provided at one grip piece 32, among the pair of grip pieces 31 and 32. Details of the stress concentration structure 37 will be described later.

As shown in Fig. 5, the ultrasonic oscillator unit 16 is configured by laminating the ultrasound oscillator 41, a backing material layer 42, and an acoustic matching layer 43.

The ultrasound oscillator 41 is composed of a piezoelectric material 44 (also referred to as a piezoelectric element) and electrodes 46 and 47. The piezoelectric material 44 is formed in a plate shape. The electrodes 46 and 47 are formed in a plate shape thinner than the piezoelectric material 44 and are laminated on both surfaces of the piezoelectric material 44. The ultrasound oscillator 41 is disposed in parallel with the grip surfaces 31A and 32A of the grip pieces 31 and 32. That is, the ultrasound oscillator 41 is disposed at a position facing the biological tissue, such as a blood vessel gripped by the grip part 15. In addition, a direction in which the ultrasound oscillator 41 oscillates is parallel to a direction in which the piezoelectric material 44 and the electrodes 46 and 47 are laminated. Accordingly, a direction of ultrasonic oscillation by the ultrasound oscillator 41 is parallel to a gripping direction Y in which the grip part 15 grips.

As the piezoelectric material 44, lead zirconate titanate (PZT) is preferably used. The piezoelectric material 44 is not limited thereto, and it is preferable to use a piezoelectric material having a high Curie point, and a piezoelectric material having a Curie point of 200°C or more, for example, lithium niobate (LiNbO₃) or barium titanate (BaTiO₃) may be used. The Curie point is also referred to as a Curie temperature, is a temperature at which a crystal structure changes from a tetragonal crystal to a cubic crystal in a case where the temperature of the piezoelectric material is raised, and is a temperature at which the piezoelectric material loses piezoelectricity (can also be referred to as a heat-resistant temperature of the piezoelectric material).

In addition, the electrodes 46 and 47 are connected to the cable 17 via the signal cable (not shown) or the like. The signal cable is wired, for example, along an inner peripheral surface or an outer peripheral surface of the insertion part 14. In a case where the ultrasonic drive unit 12 is connected to the cable 17, the electrodes 46 and 47 are electrically connected to the ultrasonic drive unit 12 via the cable 17 or the like. One of the electrodes 46 and 47 is connected to the ground via the cable 17 or the like, and the other is supplied with power of an alternating current voltage signal to be described later from the ultrasonic drive unit 12.

The acoustic matching layer 43 is provided in order to match acoustic impedance between the body of the patient and the ultrasound oscillator 41. The acoustic matching layer 43 is disposed outside the ultrasound oscillator 41, and in a strict sense, as shown in Fig. 6, is superimposed on a side facing the biological tissue gripped by the grip part 15 with respect to the ultrasound oscillator 41. That is, the acoustic matching layer 43 is provided at a position exposed from each of the grip surfaces 31A and 32A of the grip pieces 31 and 32.

As the acoustic matching layer 43 is provided, it is possible to increase light transmittance of ultrasound waves. As a material for the acoustic matching layer 43, various organic materials, in which a value of the acoustic impedance is closer to the value of the body of the patient than the piezoelectric material 44 of the ultrasound oscillator 41, can be used. Specific examples of a material for the acoustic matching layer 43 include an epoxy-based resin, a silicon rubber, polyimide, and polyethylene. In addition, the acoustic matching layer 43 is formed of a plurality of layers, and a material and the number of layers to be composed are selected as appropriate according to the value of the required acoustic impedance. The ultrasonic oscillator unit 16 may have a configuration where the acoustic matching layer 43 is not provided.

The backing material layer 42 supports the ultrasound oscillator 41 from a back side (a side opposite to the acoustic matching layer 43). A backing material is composed of, for example, a material having stiffness or the like, such as hard rubber. In addition, an air gap layer 48, that is, a gap interposed between the backing material layer 42 and the ultrasound oscillator 41 is formed between the backing material layer 42 and the ultrasound oscillator 41. The air gap layer 48 has a function of reflecting ultrasound waves emitted from the back side of the ultrasound oscillator 41 since air inside therein can reflect the ultrasound waves. Accordingly, ultrasonic oscillation can be efficiently transmitted to the biological tissue such as a blood vessel V. The present invention is not limited thereto, and a material that reflects the ultrasound waves may fill the backing material layer 42, without providing the air gap layer 48.

The stress concentration structure 37 is a protrusion that protrudes from the grip surface 32A. As described above, the acoustic matching layer 43 of the ultrasonic oscillator unit 16 is provided on the grip surface 32A. For this reason, the stress concentration structure 37 is fixed to a surface of the acoustic matching layer 43 with, for example, an adhesive. As a method of fixing the stress concentration structure 37 to the acoustic matching layer 43, for example, the stress concentration structure 37 is fixed by applying the adhesive between the acoustic matching layer 43 and the stress concentration structure 37. Alternatively, a groove may be formed in the surface of the acoustic matching layer 43, and the stress concentration structure 37 may be fitted into the groove. Alternatively, the stress concentration structure 37 may be fixed to the acoustic matching layer 43 through both bonding and fitting into the groove. The present invention is not limited thereto, and for example, the acoustic matching layer 43 and the stress concentration structure 37 may be integrally formed of a metal or the like. In addition, a configuration where the stress concentration structure 37 that is positioned at a central portion of the grip surface 32A and the ultrasonic oscillator units 16 that are adjacent to each of the right and left of the stress concentration structure 37 and that pinch the stress concentration structure 37 are further comprised may be adopted.

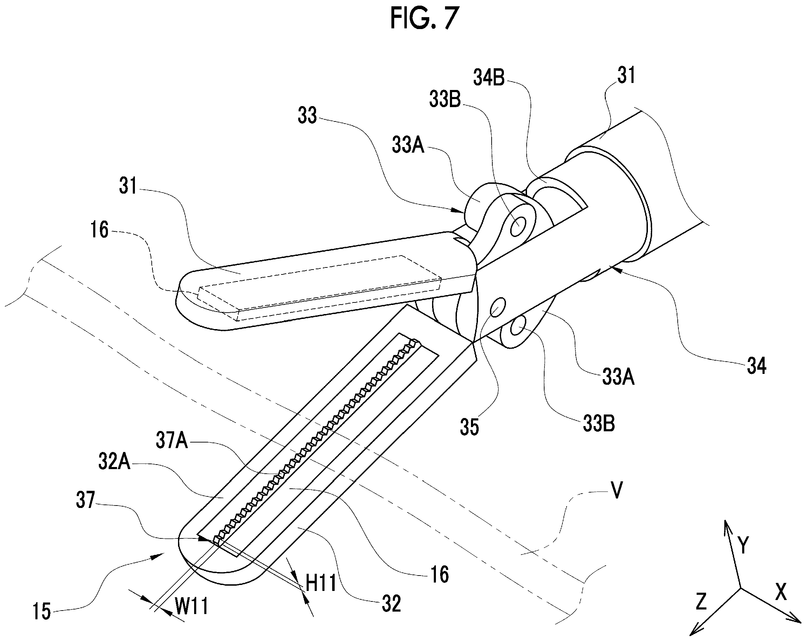

As shown in Fig. 7, the stress concentration structure 37 is a long protrusion disposed along a longitudinal direction of the grip piece 32, that is, the insertion direction Z. The stress concentration structure 37 is formed such that a distal end part 37A from the grip surface 32A in a protruding direction, that is, the gripping direction Y, is in a continuous sawtooth shape. By comprising such a stress concentration structure 37, stress is concentrated on a portion of the biological tissue gripped by the grip part 15, which is in contact with the stress concentration structure 37, and the stress is more likely to be concentrated by making the distal end part 37A in a sawtooth shape. Accordingly, even in a case where the ultrasonic treatment tool is operated with a smaller force than in a case where the ultrasonic treatment tool of the related art is operated, the blood vessel V or the like, which is the biological tissue, can be incised. In addition, as the continuous sawtooth-shaped stress concentration structure 37 is included, the grip part 15 can reliably grip the biological tissue.

The stress concentration structure 37 has hardness of 40 HBW or more in Brinell hardness and is formed of, for example, a metal, ceramics, or the like. Accordingly, stress is more likely to be further transmitted to the biological tissue gripped by the grip part 15, and thereby treatment of incising the biological tissue can be performed more efficiently. In addition, the stress concentration structure 37 has, for example, a width W11 (a dimension in the width direction X) of 0.2 mm and a height H11 (dimension in the gripping direction Y) that protrudes from the grip surface 32A of 0.2 mm (see Fig. 6).

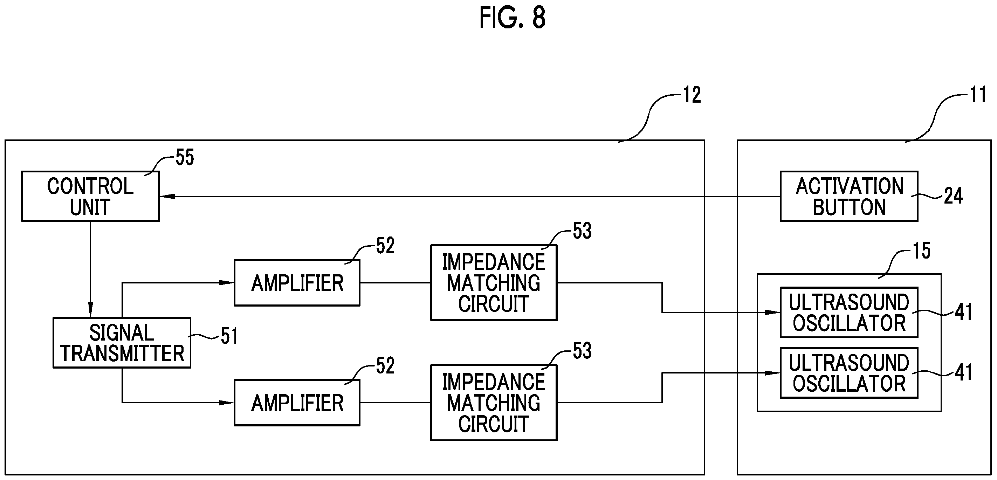

As shown in Fig. 8, the ultrasonic drive unit 12 comprises a signal transmitter 51, an amplifier 52, an impedance matching circuit 53, and the control unit 55. The signal transmitter 51 has a function of generating an alternating current voltage signal having any frequency and waveform and has, for example, the same configuration and function as a known function generator.

A program related to various types of processing is stored in a program memory (not shown) of the ultrasonic drive unit 12. The control unit 55 composed of a processor realizes functions of the signal transmitter 51, the amplifier 52, and the impedance matching circuit 53 by executing the program in the program memory.

The signal transmitter 51 outputs, for example, an alternating current voltage signal having a waveform of a sin wave. In order to supply power to a pair of ultrasound oscillators 41, the amplifier 52 and the impedance matching circuit 53 are provided for each of the ultrasound oscillators 41. The signal transmitter 51 outputs each of alternating current voltage signals having the same frequency and the same waveform to the amplifier 52. The amplifier 52 amplifies the alternating current voltage signal output from the signal transmitter 51 to a voltage level at which the ultrasound oscillator 41 can be driven. The impedance matching circuit 53 is connected in series with the amplifier 52 and can match input impedance of the alternating current voltage signal output from the amplifier 52 with impedance of the ultrasound oscillator 41.

The signal transmitter 51 outputs an alternating current voltage signal at a frequency at which a difference from the resonance frequency of the piezoelectric material 44 is within 20% under the control of the control unit 55. The piezoelectric material 44 has a resonance frequency corresponding to a thickness dimension D1 (see Figs. 5 and 6), and specifically, in a case where the thickness dimension is defined as D1 (m), the resonance frequency corresponding to the thickness dimension D1 is defined as f (Hz), and the sound speed of the piezoelectric material 44 (the speed of sound waves transmitted through the piezoelectric material 44) is defined as v (m/sec), a relationship of D1 = v/2f is established.

In addition, the control unit 55 controls the signal transmitter 51 so that an alternating current voltage signal for driving the ultrasound oscillator 41 is continuously supplied. The term "continuously supplied" herein means that the alternating current voltage signal is continuously output from the signal transmitter 51 without interruption at least during the driving of the ultrasound oscillator 41.

In addition, the control unit 55 receives an activation signal transmitted from the activation button 24. The control unit 55 that has received the activation signal controls the signal transmitter 51 so that an alternating current voltage signal for driving the ultrasound oscillator 41 is supplied.

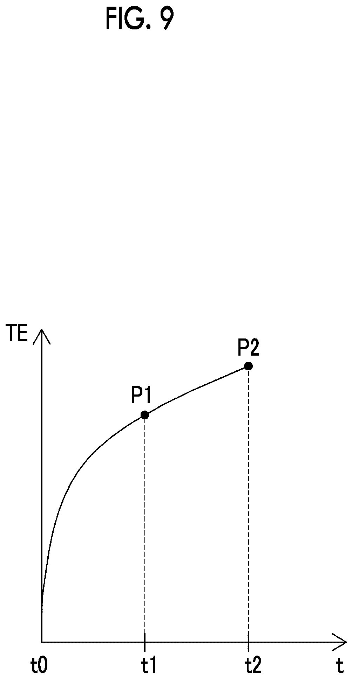

Fig. 9 is a graph showing ultrasonic oscillation and a temperature rise attributable to the device driving, that is, a relationship between a driving time t of the ultrasound oscillator 41 and a blood vessel temperature TE in a case where the blood vessel V is gripped by the grip pieces 31 and 32 and the ultrasound oscillator 41 is driven. P1 and P2 indicate completion points at which sealing and incision are completed in a case where ultrasonic oscillation and heat attributable to the device driving are transmitted to the blood vessel V, respectively. In addition, t0 is a driving time in a case where the ultrasound oscillator 41 starts the ultrasonic oscillation, and t1 and t2 show driving times when the sealing and incision of the blood vessel V are completed, respectively. As shown in the graph, the temperature of the blood vessel V, which is gripped by the grip part 15 and to which the ultrasonic oscillation and the heat attributable to the device driving are transmitted, rises, and the sealing is completed in a case where the driving time t1 is reached. Further, in a case where the driving time t1 has passed and the driving time t2 is reached, the incision is completed.

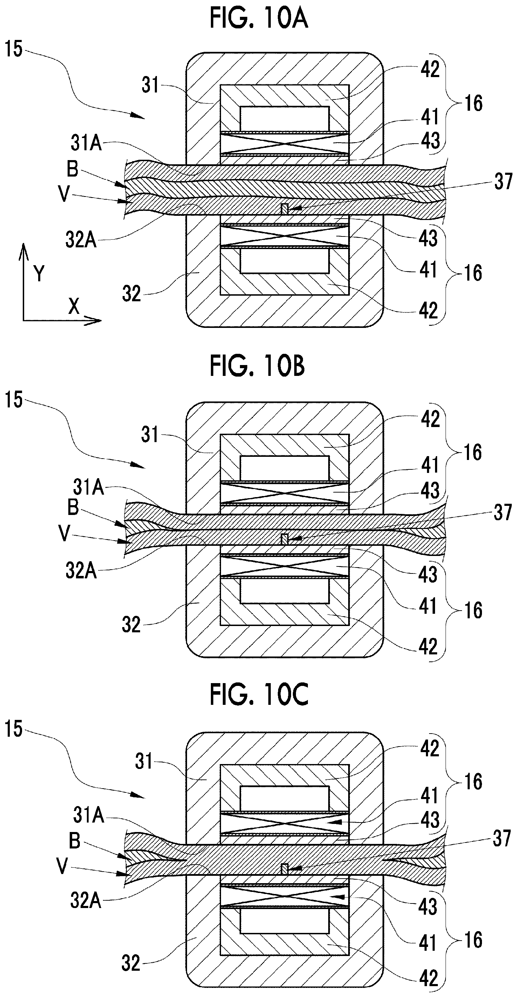

An operation in a case where a doctor who is the user performs sealing and incision by the ultrasonic treatment tool 11 by using the ultrasonic treatment tool system 10 will be described with reference to Figs. 10A to 11C. Figs. 10A to 11C show examples of treating the blood vessel V as the biological tissue. In addition, the reference numeral B indicates blood present in the blood vessel V. Before performing treatment with the ultrasonic treatment tool 11, the doctor can puncture the blood vessel V in the patient's body with the trocar at a desired position and depth and insert the ultrasonic treatment tool 11 via the trocar, so that the grip part 15 can reach the blood vessel V.

The doctor swings the trigger 23 in the direction separated away from the grip 22 with the finger put thereon while observing an endoscope image captured by an endoscope inserted into the patient's body, for example, from a position different from the ultrasonic treatment tool 11, bringing the grip pieces 31 and 32 of the grip part 15 into an open state. Accordingly, positions of the pair of the grip pieces 31 and 32 can be aligned at positions of pinching the blood vessel V (a state shown in Fig. 7).

Next, as shown in Fig. 10A, the doctor swings the trigger 23 in a direction of pulling to the grip 22 side so that the grip pieces 31 and 32 are rotationally moved from an open state to a closed state. Accordingly, the grip part 15 grips the blood vessel V Figs. 10A to 10C are explanatory views showing processes in a case where treatment of sealing the blood vessel V is performed. Then, the doctor operates the activation button 24 to be pressed while maintaining a state where the grip pieces 31 and 32 grip the blood vessel V. An activation signal transmitted in response to the pressing operation of the activation button 24 is input into the control unit 55 of the ultrasonic drive unit 12 via the cable 17 or the like.

The control unit 55 into which an activation signal is input activates each unit of the ultrasonic drive unit 12 and controls the signal transmitter 51 so that an alternating current voltage signal for driving the ultrasound oscillator 41 is continuously supplied. Accordingly, the driving of the ultrasound oscillator 41 is started, and ultrasonic oscillation is transmitted to the blood vessel V.

Fig. 10B is a state immediately after ultrasonic oscillation of the ultrasound oscillator 41 is started to be transmitted to the blood vessel V while a state where the grip pieces 31 and 32 grip the blood vessel V is maintained. In this state, the blood vessel V is not yet sealed. In this state, it is preferable that a gripping pressure at which the grip pieces 31 and 32 grip the blood vessel V is 2 MPa or less. Since the grip part 15 comprises the stress concentration structure 37, in a case where the grip pieces 31 and 32 grip the blood vessel V, the stress concentration structure 37 can reliably grip the blood vessel V by biting into the blood vessel V. That is, treatment of sealing the blood vessel V can be efficiently performed.

Then, as shown in Fig. 10C, the temperature of the blood vessel V rises by transmitting ultrasonic oscillation of the ultrasound oscillator 41 while a state where the grip pieces 31 and 32 grip the blood vessel V is maintained. In a case where the ultrasound oscillator 41 is driven at a frequency at which a difference from the resonance frequency described above is within 20%, the temperature of the blood vessel V reaches 150°C or more within 5 seconds. In this case, both heat generation of the ultrasound oscillator 41 itself and heat generation caused by ultrasonic oscillation raise the temperature of the blood vessel V.

In a case where the blood vessel V has reached a certain temperature (approximately 60°C to 90°C) or more, components such as proteins are coagulated. For this reason, a portion of the blood vessel V, which is gripped by the grip pieces 31 and 32, is sealed. In this case, the blood vessel V is gripped for a certain time or less when the incision of the blood vessel V is not started and at a sealing pressure to an extent that blood B in the blood vessel V does not leak. The certain time and the sealing pressure depend on mechanical properties of the blood vessel V and a gripping pressure immediately after ultrasonic oscillation is started to be transmitted to the blood vessel V.

In a case where treatment of the blood vessel V is performed under the conditions described above, the blood vessel V is completely sealed at the driving time t1 = 5 seconds and is completely incised at the driving time t2 = 10 seconds (see Fig. 9). Accordingly, in a case where treatment of sealing the blood vessel V is performed, it is preferable that the treatment is terminated in 5 seconds or more and less than 10 seconds after ultrasonic oscillation of the ultrasound oscillator 41 is started to be transmitted.

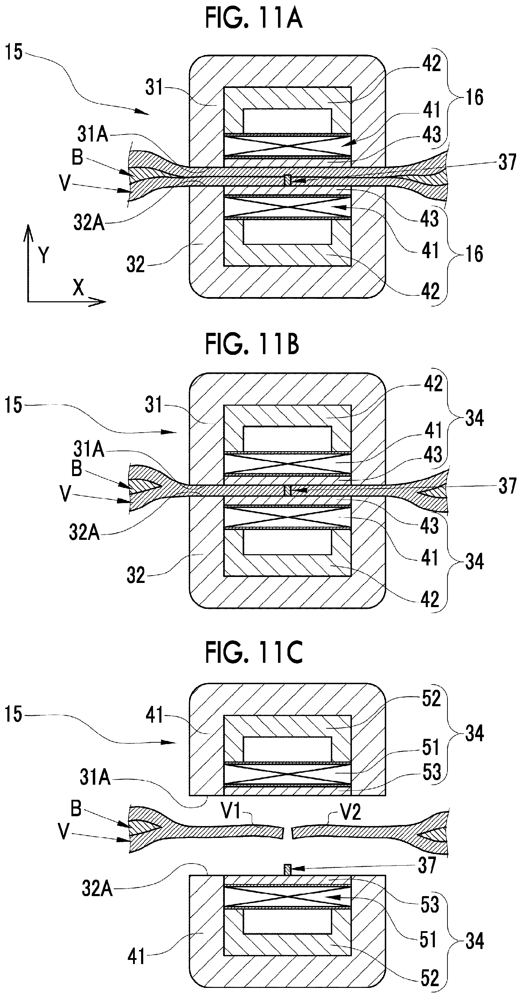

On the other hand, in a case where treatment of incising the blood vessel V is performed, the doctor grips the blood vessel V by operating the ultrasonic treatment tool 11 as in a case where treatment of sealing the blood vessel V is performed (see Fig. 11A). Figs. 11A to 11C are explanatory views showing processes in a case where treatment of incising the blood vessel V is performed. Then, the doctor operates the activation button 24 to be pressed while maintaining a state where the grip pieces 31 and 32 grip the blood vessel V. An activation signal transmitted in response to the pressing operation of the activation button 24 is input into the control unit 55 of the ultrasonic drive unit 12.

The control unit 55 into which an activation signal is input activates each unit of the ultrasonic drive unit 12 and controls the signal transmitter 51 so that an alternating current voltage signal is continuously supplied. Accordingly, the driving of the ultrasound oscillator 41 is started, and ultrasonic oscillation is transmitted to the blood vessel V. The doctor increases a gripping pressure at which the blood vessel V is gripped by further swinging the trigger 23 in the direction of pulling to the grip 22 side. In this case, the gripping pressure is larger than a case of performing treatment of sealing the blood vessel V.

As shown in Fig. 11B, in a case where the temperature of the blood vessel V rises as ultrasonic oscillation of the ultrasound oscillator 41 and heat attributable to the device driving are transmitted, and a gripping pressure of the grip part 15 further increases, stress is concentrated on the portion of the blood vessel V, which is in contact with the stress concentration structure 37. In addition, in this case, the blood vessel V has decreased mechanical strength due to the temperature rise. Since the stress concentration structure 37 is a protrusion that protrudes from the grip surface 32A, the portion of the blood vessel V, which is in contact with the stress concentration structure 37, has a smaller thickness than a portion in contact with the grip surface 32A. Further, as the distal end part 37A has a sawtooth shape, stress is likely to be concentrated. Accordingly, the blood vessel V can be easily incised even in a case where the ultrasonic treatment tool 11 is operated with a smaller force than in a case of operating the ultrasonic treatment tool of the related art. That is, treatment of incising the blood vessel V can be efficiently performed.

In a case where the blood vessel V is treated under the conditions described above, the blood vessel V is completely incised at the driving time t2 = 7 seconds. Accordingly, in a case where treatment of incising the blood vessel V is performed, it is preferable that the treatment is terminated after 7 seconds or more from the start of transmission of ultrasonic oscillation of the ultrasound oscillator 41.

After the driving time t2 when the incision is completed has passed, as shown in Fig. 11C, the doctor swings the trigger 23 in the direction separated away from the grip 22, bringing the grip pieces 31 and 32 of the grip part 15 into an open state. Incised portions V1 and V2 of the blood vessel V are separated from each other and are separated from the grip pieces 31 and 32. In this manner, treatment of incising the blood vessel V is terminated.

### [Second Embodiment]

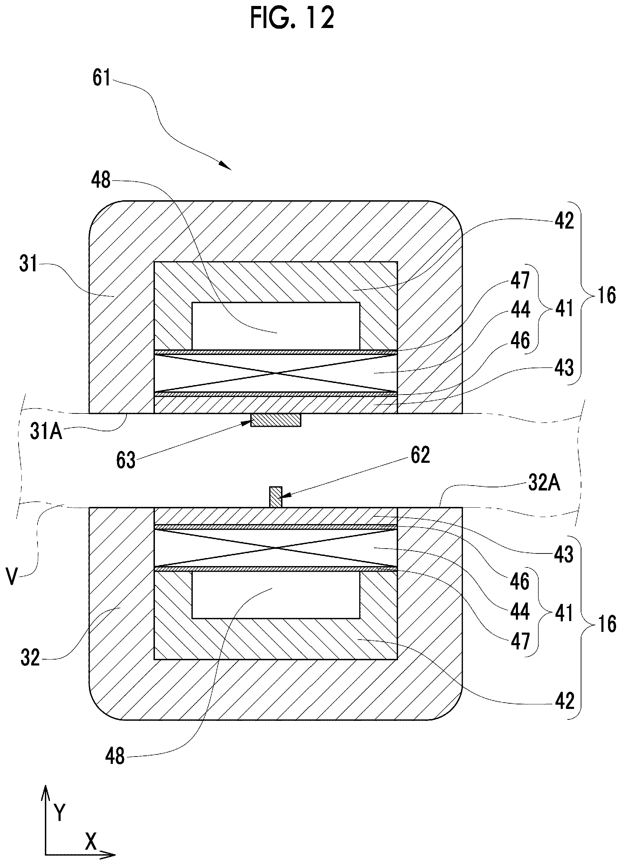

As shown in Figs. 12 to 14, in addition to the configuration of the first embodiment, a surface facing the stress concentration structure may comprise a member having the same hardness as the stress concentration structure. In this case, it is preferable that a grip part 61 comprises a stress concentration structure 62 at one grip piece 32 of the pair of grip pieces 31 and 32 and comprises a facing member 63 that has the same hardness as the stress concentration structure 62 at the other grip piece 31. Hereinafter, the same components as the grip part 15 in the first embodiment will be assigned with the same reference numerals, and description thereof will be omitted. In addition, the configuration is the same as that of the ultrasonic treatment tool 11 of the first embodiment, except that the configuration of the grip part 15 is replaced with the configuration of the grip part 61, and description thereof will be omitted.

As shown in Fig. 12, the stress concentration structure 62 is a protrusion that protrudes from the grip surface 32A and is fixed to the surface of the acoustic matching layer 43, like the stress concentration structure 37 in the first embodiment. In addition, the stress concentration structure 62 has hardness of 40 HBW or more in Brinell hardness and is formed of, for example, a metal, ceramics, or the like. A method of fixing the stress concentration structure 62 to the acoustic matching layer 43 is the same as the method of fixing the stress concentration structure 37 to the acoustic matching layer 43 in the first embodiment. The present invention is not limited thereto, and for example, the acoustic matching layer 43 and the stress concentration structure 62 may be integrally formed of a metal or the like.

The stress concentration structure 62 is a long protrusion disposed along the longitudinal direction of the grip piece 32, that is, the insertion direction Z. The stress concentration structure 62 is formed such that a distal end part 62A from the grip surface 32A in the protruding direction, that is, the gripping direction Y, is in a continuous sawtooth shape, like the stress concentration structure 37 in the first embodiment.

The facing member 63 is a plate-shaped member disposed at a position facing the stress concentration structure 62. The facing member 63 is formed in a long shape disposed along a longitudinal direction of the grip piece 31. The facing member 63 is at the grip surface 31A of the grip piece 31 and is fixed to the surface of the acoustic matching layer 43. The facing member 63 has the same hardness as the stress concentration structure 62 as described above, and specifically, a surface facing the stress concentration structure 62 has hardness of 40 HBW or more in Brinell hardness and is formed of, for example, metal, ceramics, or the like.

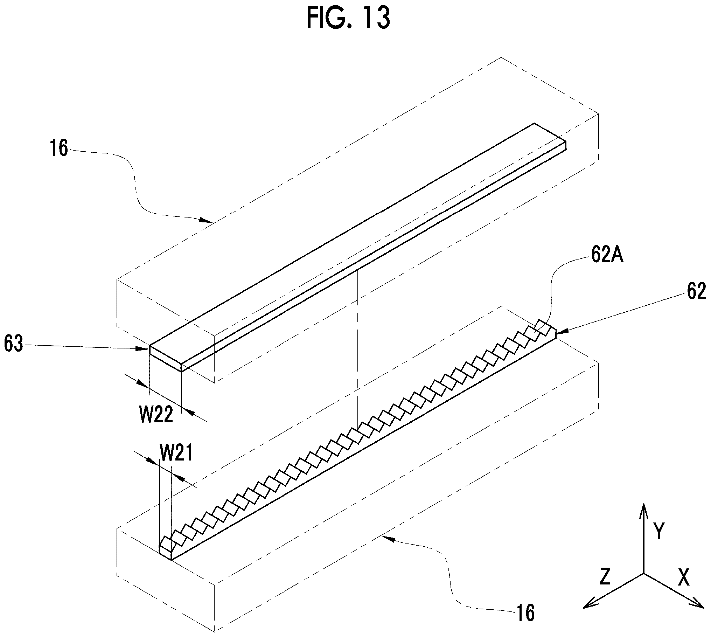

As shown in Fig. 13, a width W22 (a dimension in the width direction X) of the facing member 63 is larger than a width W21 of the stress concentration structure 62. For example, the width W22 of the facing member 63 is 0.6 mm, and the width W21 of the stress concentration structure 62 is 0.2 mm. Accordingly, in a case where the grip part 15 is closed, the stress concentration structure 62 and the facing member 63 can be reliably faced with each other.

An operation in a case where the doctor who is the user performs incision treatment with the ultrasonic treatment tool according to the present embodiment will be described with reference to Figs. 12 to 14. The operation in a case of performing sealing treatment with the ultrasonic treatment tool of the present embodiment is the same as in the first embodiment, and description thereof will be omitted.

In a case where treatment of incising the blood vessel V is performed, the doctor grips the blood vessel V by operating the ultrasonic treatment tool 11, as in the case of the first embodiment (a state shown in Fig. 12). Then, the doctor operates the activation button 24 to be pressed while maintaining a state where the grip pieces 31 and 32 grip the blood vessel V. An activation signal transmitted in response to the pressing operation of the activation button 24 is input into the control unit 55.

The control unit 55 into which an activation signal is input controls the signal transmitter 51 so that an alternating current voltage signal is continuously supplied. Accordingly, the driving of the ultrasound oscillator 41 is started, and ultrasonic oscillation is transmitted to the blood vessel V. The doctor increases a gripping pressure at which the blood vessel V is gripped by further swinging the trigger 23 in the direction of pulling to the grip 22 side.

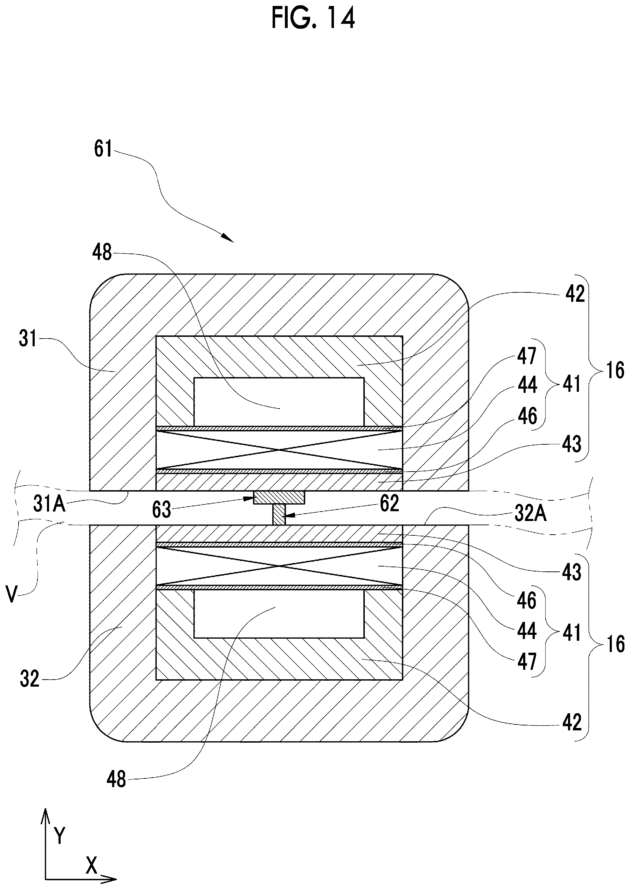

As shown in Fig. 14, in a case where the temperature of the blood vessel V rises as ultrasonic oscillation of the ultrasound oscillator 41 is transmitted, and a gripping pressure of the grip part 61 further increases, stress is concentrated on a portion of the blood vessel V, which is in contact with the stress concentration structure 62. In the present embodiment, the facing member 63 having the same hardness is provided at a position facing the stress concentration structure 62 (see Fig. 13). Since the blood vessel V is in a state of being pinched between the stress concentration structure 62 and the facing member 63, stress is not dispersed. Accordingly, the stress is more likely to be concentrated on the portion of the blood vessel V gripped by the grip part 61, which is in contact with the stress concentration structure 62. Accordingly, the blood vessel V can be easily incised even in a case where the ultrasonic treatment tool 11 is operated with a force smaller than in the first embodiment. That is, treatment of incising the blood vessel V can be efficiently performed.

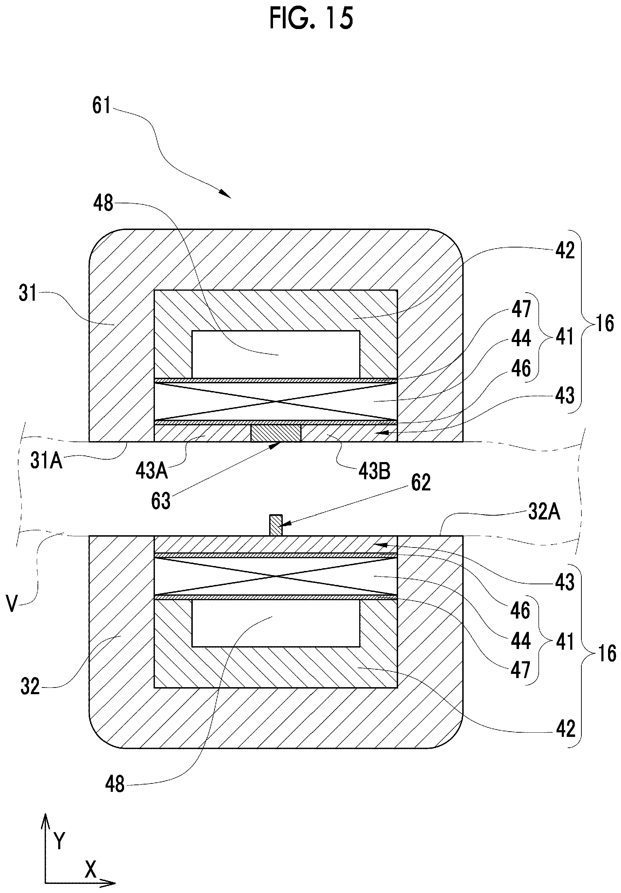

The facing member 63 comprised in the other grip piece 31 is configured to be fixed to the ultrasonic oscillator unit 16 in the second embodiment, but without being limited thereto, may constitute a part of the ultrasonic oscillator unit 16. In a modification example shown in Fig. 15, for example, a part of the acoustic matching layer 43 constituting the grip surface 31A in the ultrasonic oscillator unit 16 is defined as the facing member 63 and is made of a material having the same hardness as the stress concentration structure 62. In this case, since the facing member 63 is disposed at the center of the acoustic matching layer 43, a part excluding the facing member 63 is divided into two acoustic matching layers 43A and 43B. The acoustic matching layers 43A and 43B have the same materials and structures as in the respective embodiments. It is preferable that the acoustic matching layers 43A and 43B and the facing member 63 are fixed to each other and are disposed on the grip surface 31A.

Although treatment of gripping the blood vessel as the biological tissue and sealing and incising the blood vessel has been described as an example in each of the embodiments, the biological tissue to be treated is not limited to the blood vessel, and can also be applied to a tubular biological tissue, for example, a part of the intestine, a part of the reproductive system, or the like.

Although the ultrasound oscillator 41 is comprised in both the pair of grip pieces 31 and 32 constituting the grip part 15, and ultrasonic oscillation is transmitted to the structure from both the grip pieces 31 and 32 in the embodiment, the present invention is not limited thereto, and the ultrasound oscillator 41 may be provided at only one of the pair of grip pieces 31 and 32 constituting the grip part 15. In this case, it is preferable that the ultrasound oscillator 41 is disposed at one grip piece, and the grip surface of the other grip piece facing the ultrasound oscillator 41 is formed of a material that reflects ultrasonic oscillation caused by the ultrasound oscillator 41. In addition, in this case, it is preferable that the stress concentration structure described in each of the embodiments described above is provided at one of the pair of grip pieces 31 and 32 where the ultrasound oscillator 41 is disposed.

### Explanation of References

10: ultrasonic treatment tool system
11: ultrasonic treatment tool
12: ultrasonic drive unit
13: operating part
14: insertion part
15: grip part
16: ultrasonic oscillator unit
17: cable
21: operating part main body
22: grip
23: trigger
24: activation button
26: operation wire
31, 32: grip piece
31A, 32A: grip surface
33: link mechanism
33A: link plate
33B: connecting pin
33C: fitting pin
34: support member
34A: through-hole
34B: notch
35: support shaft
36: connecting member
37: stress concentration structure
37A: distal end part
41: ultrasound oscillator
42: backing material layer
43: acoustic matching layer
43A, 43B: acoustic matching layer
44: piezoelectric material
46, 47: electrode
48: air gap layer
51: signal transmitter
52: amplifier
53: impedance matching circuit
55: control unit
61: grip part
62: stress concentration structure
62A: distal end part
63: facing member
B: blood
D1: thickness dimension
H11: height
t, t0, 11, t2: driving time
TE: temperature
V: blood vessel
V1, V2: incised portion
W11: width
W21: width
W22: width

## Claims

1. An ultrasonic treatment tool comprising:
a grip part that is provided at a distal end part and that grips a biological tissue in a subj ect;
an ultrasound oscillator that transmits ultrasonic oscillation to the biological tissue gripped by the grip part; and
a stress concentration structure that is provided at the grip part and that protrudes from a grip surface facing the biological tissue,
wherein the stress concentration structure is a long protrusion disposed along a longitudinal direction of the grip part, and
a distal end part from the grip surface in a protruding direction is formed in a continuous sawtooth shape.

2. The ultrasonic treatment tool according to claim 1,
wherein the ultrasound oscillator is disposed at the grip part.

3. The ultrasonic treatment tool according to claim 1 or 2,
wherein the stress concentration structure has hardness of 40 HBW or more in Brinell hardness.

4. The ultrasonic treatment tool according to claim 3,
wherein a surface of the grip part, which faces the stress concentration structure, has hardness of 40 HBW or more in Brinell hardness.

5. The ultrasonic treatment tool according to any one of claims 1 to 4,
wherein the ultrasound oscillator is composed of a piezoelectric material and is driven by an alternating current voltage signal having a frequency at which a difference from a resonance frequency corresponding to a thickness dimension of the piezoelectric material is within 20%.

6. The ultrasonic treatment tool according to claim 5,
wherein the piezoelectric material is lead zirconate titanate.

7. The ultrasonic treatment tool according to claim 5,
wherein a Curie point of the piezoelectric material is 200°C or more.

8. The ultrasonic treatment tool according to any one of claims 1 to 7,
wherein an alternating current voltage signal for driving the ultrasound oscillator is continuously supplied.

9. The ultrasonic treatment tool according to any one of claims 1 to 8,
wherein the grip part grips a blood vessel which is the biological tissue.

10. The ultrasonic treatment tool according to any one of claims 1 to 9, which is a surgical treatment tool comprising an insertion part that is installed consecutively to a base end of the grip part and an operating part that is installed consecutively to a base end of the insertion part.

11. The ultrasonic treatment tool according to any one of claims 1 to 10,
wherein the grip part comprises a pair of grip pieces that grips the biological tissue, and the stress concentration structure is provided at one grip piece.
